# EUROPÄISCHE PATENTANMELDUNG

(11) **EP 2 893 866 A1**
(43) Veröffentlichungstag der Anmeldung: **15.07.2015**
(21) Anmeldenummer: 14156160.5
(22) Anmeldetag: 21.02.2014
(51) Int. Cl.: A61B 1/12, A61L 2/235

(54) **Stethoskopreinigungsvorrichtung**

(30) Priorität: 14.01.2014 WO PCT/CN2014/070217
(71) Anmelder: Joker AG, 3210 Kerzers/FR (CH)
(72) Erfinder: Flury, Meinrad, 3210 Kerzers/FR (CH)
(74) Vertreter: Schneider Feldmann AG Patent- und Markenanwälte

(57) **Zusammenfassung**

Eine Stethoskopreinigungsvorrichtung (1) zur Reinigung und Desinfektion von Stethoskopen hat einen Aufnahmebehälter (3) mit einer Aufnahmeöffnung (4), in dem eine Reinigungspatrone (2) auswechselbar gehalten ist. Die Reinigungspatrone (2) enthält eine Reinigungsmasse die als Reinigungsgel ausgebildet ist. Dabei ist ein erstes an der Reinigungspatrone (2) angebrachtes Adapterteil (7) mit einem aufsetzbaren zweiten Adapterteil (8) einrastend verbindbar. Das zweite Adapterteil (8) ist seinerseits mit dem Aufnahmebehälter (3) einrastend verbindbar. Zudem weist das zweite Adapterteil (8) eine Lippendichtung (9) aus einem elastischen Material auf.

## Beschreibung

Die Erfindung betrifft eine Stethoskopreinigungsvorrichtung nach Patentanspruch 1.

Stethoskope sind sehr häufig verwendete und tragbare Diagnosegeräte für Ärzte. Üblicherweise werden solche Stethoskope bei einer ganzen Reihe von verschiedenen Patienten eingesetzt, oft sogar ohne dass zwischen den einzelnen Untersuchungen eine gründliche Reinigung durchgeführt wird. Versäumt man es jedoch solche Reinigungen und Desinfektionen durchzuführen, so kann es geschehen, dass das Stethoskop mit pathogenen Mikroorganismen kontaminiert wird. Fortgesetzter Gebrauch kann dann zu iatrogenen Infektionen führen, also solchen, die durch die ärztliche Behandlung erst ausgelöst werden.

Man hat diese Zusammenhänge natürlich erkannt und es sind auch Vorrichtungen bekannt, die dem medizinischen Personal helfen sollen, iatrogene Infektionen zu vermeiden.

Ein Beispiel einer solchen Vorrichtung ist eine Stethoskopreinigungsvorrichtung mit der Produktbezeichnung "cleanstethoscope" der Firma Cleanint. Es handelt sich dabei um einen einfachen am Arztkittel befestigbaren Halter mit einem Schwamm, der mit einer antimikrobiellen Substanz durchtränkt ist. Ein Stethoskop kann auf leicht zugängliche Weise nach jedem Gebrauch in diesem Halter eingehängt werden und die Kontaktfläche des Stethoskops gelangt dann in Berührung mit dem Schwamm. Auf diese Weise erfolgt die Desinfektion der mit der Haut jedes Patienten in Berührung kommenden Kontaktfläche des Stethoskops. Nachteilig an dieser Lösung ist, dass jeweils nur Teile des Stethoskops desinfiziert werden.

Ein weiteres Beispiel einer Reinigungsvorrichtung für ein medizinisches Gerät das wiederholt zur Anwendung gelangt und bei dem die Infektionsgefahr ohne Zwischenreinigung sogar noch grösser ist, ist aus der WO-2013/056971 bekannt. Es handelt sich dabei um ein Interdentalzahnbürstenreinigungsmittel bei dem ein Reinigungsgel zur Anwendung gelangt. Ein solches Reinigungsgel ist eine leicht knet- und formbare Masse mit antiseptischer und auch reinigender und desinfizierender Wirkung, das heisst mit entsprechend wirkenden Zusatzstoffen, wobei die zu reinigenden Interdentalbürsten zur Reinigung für eine gewisse Zeit in das Reinigungsgel eingetaucht oder eingesteckt werden. Die Reinigungswirkung beruht darauf, dass sich die weiche und leicht verformbare Masse des Reinigungsgels sehr leicht an jede Kontur eines eingetauchten Gegenstandes anpasst und infolge eines Membraneffektes Verunreinigungen und Partikel an der Oberfläche des eingetauchten Gegenstandes in sich aufnimmt und einschliesst und so vom eingetauchten Gegenstand entfernt. Derartige Reinigungsmassen sind bekannt, beispielsweise aus der WO-2011/134715, und sie haben grundsätzlich stark kohäsive Eigenschaften, d.h. auch bei starken Verformungen bleiben keine Rückstände an den zu reinigenden Oberflächen hängen.

Das bekannte Interdentalzahnbürstenreinigungsmittel aus der WO-2013/056971 umfasst eine Haltevorrichtung mit Aufnahmeöffnungen, in der zwei jeweils eine Reinigungsmasse enthaltende und mit Kappen versehene Behälter auswechselbar gehalten sind. In Einführungsöffnungen der Behälter sind die zu reinigenden Interdentalbürsten einführbar und können so in Wirkverbindung mit dem in den Behältern vorhandenen Reinigungsgel gebracht werden.

Die Aufgabe der Erfindung besteht darin, eine Weiterentwicklung einer tragbaren Reinigungsvorrichtung mit einer Gelmasse zur Verfügung zu stellen, die für Stethoskope geeignet ist und die einen einfachen und praktikablen Gebrauch von Reinigungspatronen erlaubt.

Diese Aufgabe wird durch die Merkmalskombination des Patentanspruchs 1 gelöst.

Die Lösung besteht darin, dass bei einer gattungsgemässen Reinigungsvorrichtung
- ein erstes an der Reinigungspatrone angebrachtes Adapterteil mit einem aufsetzbaren zweiten Adapterteil einrastend verbindbar ist, dass
- das zweite Adapterteil mit dem Aufnahmebehälter einrastend verbindbar ist, und dass
- das zweite Adapterteil eine Lippendichtung aus einem elastischen Material aufweist.

Die Kombination von erstem und zweitem Adapterteil bringt zunächst den Vorteil, dass Ersatz-Reinigungspatronen einfach aufgebaut und einfach verschliessbar sind (Zur Verwendung einer Ersatz-Reinigungspatrone muss beispielsweise lediglich eine Verschlussfolie vom ersten Adapterteil abgezogen werden). Das konstruktiv einfachere erste Adapterteil ist deshalb an der Reinigungspatrone angebracht. Das zweite Adapterteil bringt nicht nur den Vorteil, dass es leicht und ohne zusätzliche Hilfsmittel sowohl mit der Reinigungspatrone als auch mit dem Aufnahmebehälter verbindbar ist, sondern dass es integriert auch eine geeignet ausgeformte Lippendichtung enthält. Die Letztere ist im Falle von Stethoskopen, also von medizinischen Geräten die aufgrund ihrer Form beim Herausziehen aus der Reinigungsmasse dieselbe mitreissen oder wenigstens teilweise hervortreten lassen könnten, natürlich besonders wünschenswert. Die vorliegende Lösung erlaubt also, eine Lippendichtung, deren Aufgabe es ist, das Reinigungsgel in der Reinigungspatrone zurückzuhalten, gleich in einem einfachen, leicht zugänglichen und deshalb seinerseits wiederum sehr reinigungsfreundlichen Adapterteil zu integrieren. Die Reinigungspatronen selbst können, wie vorstehend erwähnt, mit dieser zweiteiligen Adapterlösung konstruktiv einfach gehalten werden und eignen sich deshalb auch gut für Vermarktungskonzepte bei denen die Rezyklierbarkeit der verwendeten Materialien angestrebt wird.

In speziell vorteilhafter Weise kann auch eine zweilagige bzw. zweiteilige Lippendichtung bestehend aus einer inneren und einer äusseren Lippendichtung verwendet werden. Mit einer zweiteiligen Lippendichtung kann die Rückhaltefähigkeit für die Reinigungsmasse und die Sauberkeit am Einführungsschlitz verbessert werden.

Besonders vorteilhaft ist auch, dass die Reinigungspatrone elastisch zusammendrückbar ist und dass der Aufnahmebehälter mindestens in einem Teilbereich ebenfalls elastisch zusammendrückbar ist. Damit gelingt es, die Reinigungs- und Desinfektionswirkung durch intensiveren Kontakt des Stethoskops mit der Reinigungsmasse noch weiter zu verbessern.

Schliesslich kann die erfindungsgemässe Stethoskopreinigungsvorrichtung mit Anzeigemitteln zur Beurteilung der Gebrauchsfähigkeit versehen sein. Derartige Anzeigemittel können im einfachsten Fall aus einem Sichtfenster bestehen, das es erlaubt, unter Verwendung eines sich verfärbenden und der Reinigungsmasse beigemischten Indikatorstoffes eine optische Beurteilung der noch verbleibenden Wirksamkeit bzw. der noch vorhandenen Reinigungsfähigkeit vorzunehmen. Die Anzeigemittel können aber auch elektronisch realisiert werden, beispielsweise mit einem Sensorelement, einer elektronischen Schaltung und einem am Aufnahmebehälter angebrachten Anzeigeelement in der Form eines alphanumerischen Displays.

Eine mögliche Ausführungsform der vorliegenden Erfindung wird im Folgenden anhand von Zeichnungen beschrieben. Dabei zeigt
- Fig. 1: die Hauptbestandteile der Stethoskopreinigungsvorrichtung,
- Fig. 2: vier Ansichten der Stethoskopreinigungsvorrichtung,
- Fig. 3: eine Explosionszeichnung der Stethoskopreinigungsvorrichtung,
- Fig. 4: einen Querschnitt durch die Stethoskopreinigungsvorrichtung,
- Fig. 5: einen Längsschnitt durch die Stethoskopreinigungsvorrichtung,
- Fig. 6: den Gebrauch der Stethoskopreinigungsvorrichtung durch Zusammendrücken,
- Fig. 7: die Funktionsweise einer inneren Lippendichtung bei eingesetztem und bei entnommenem Stethoskop, und
- Fig. 8: die prinzipielle Anordnung eines Sensorelements im Aufnahmebehälter und eines Anzeigeelements am Aufnahmebehälter.

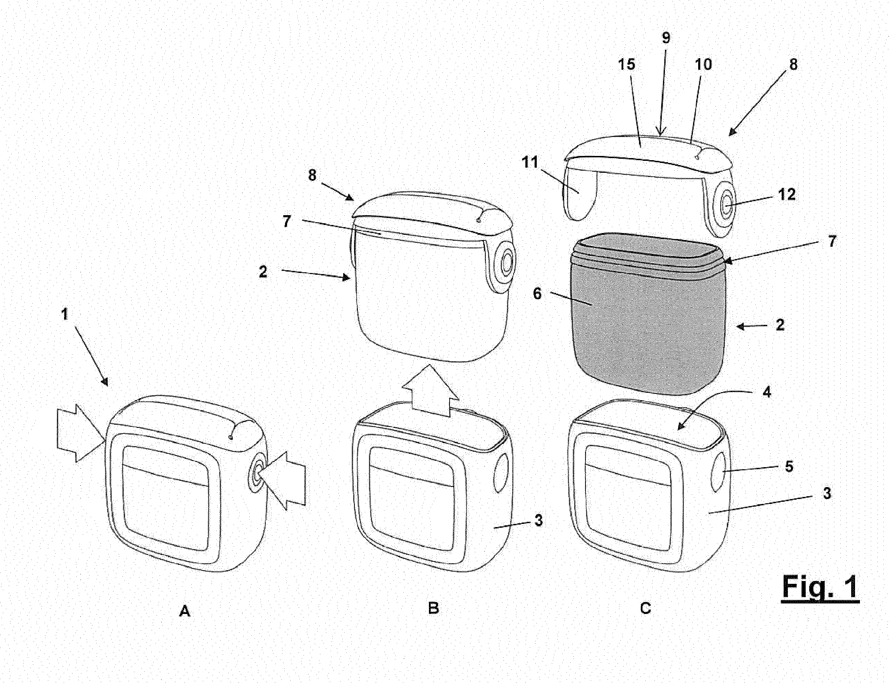

Die Fig. 1 zeigt die Hauptbestandteile der Stethoskopreinigungsvorrichtung 1 in einer funktionalen Übersicht, d.h. sie dient auch zum Verständnis und zur Veranschaulichung des Vorgangs bei einem Ersatz einer in der Stethoskopreinigungsvorrichtung auswechselbar gehaltenen Reinigungspatrone 2. Bedienung und Gebrauch der Stethoskopreinigungsvorrichtung 1 werden weiter unten noch näher beleuchtet.

Die Stethoskopreinigungsvorrichtung 1 zur Reinigung und Desinfektion von Stethoskopen umfasst einen Aufnahmebehälter 3 mit einer Aufnahmeöffnung 4, in dem eine eine Reinigungsmasse (nicht dargestellt) enthaltende Reinigungspatrone 2 auswechselbar gehalten ist, wobei die Reinigungsmasse ein Reinigungsgel ist. Der Aufnahmebehälter 3 hat weiterhin zwei seitlich angebrachte Ausnehmungen 5. Auf die Beschaffenheit des Reinigungsgels wird im Anschluss an die Beschreibung der Fig. 1 noch weiter eingegangen.

An der Reinigungspatrone 1, die ihrerseits vorzugsweise einen elastischen Gel-Behälter 6 aufweist, ist ein erstes Adapterteil 7 angebracht (vgl. dazu Teil C der vorliegenden Figur). Der Gel-Behälter 6 (grau schattiert) kann also im Prinzip auch schlauchbeutelartig gestaltet sein und eine Öffnung im ersten Adapterteil 7 ist bei der Auslieferung vorzugsweise mit einer abziehbaren Schutzfolie (nicht dargestellt) abgedeckt. Auf diese Weise ist der Inhalt der Reinigungspatrone 1 gegen vorzeitige Verunreinigung geschützt.

Ein zweites Adapterteil 8 ist einrastend, aber leicht lösbar mit dem ersten Adapterteil 7 der Reinigungspatrone 1 verbindbar (vgl. dazu Teile B und C der vorliegenden Figur). Das zweite Adapterteil 8 hat zudem eine Lippendichtung 9 aus einem elastischen Material. Die Lippendichtung 9 hat einen in Längsrichtung verlaufenden mittigen Schlitz 10 zum Einführen des zu reinigenden Stethoskops. Das zweite Adapterteil 8 hat zudem beidseits nach unten vorstehende Laschen 11 mit nach aussen vorstehenden Nocken 12. Das zweite Adapterteil 8 passt formgenau in die Aufnahmeöffnung 4 des Aufnahmebehälters 3, wobei die vorstehenden Nocken 12 in die Ausnehmungen 5 am Aufnahmebehälter 3 eingreifen und wodurch die Reinigungspatrone 1 mit dem zweiten Adapterteil 8 unverlierbar im Aufnahmebehälter 3 gehalten ist (vgl. dazu Teil A der vorliegenden Figur). Die Nocken 12 und die Ausnehmungen 5 bilden somit eine einrastende Verbindung. Die Adapterteile 7,8 sind zudem so gebaut, dass die Zusammensetzbarkeit und Lösbarkeit ohne Zuhilfenahme von Werkzeugen möglich ist.

Das Reinigungsgel selbst ist ein Adsorptionsmittel das oberflächenaktive Stoffe und andere Bestandteile enthält. Es ist darauf ausgelegt, Bakterien, Hefen, Schimmel, Milben und insbesondere auch MRSA- (Methicillin Resistant Staphylococcus Aureus) Erreger und Grippe-Viren (einschliesslich H1N1 Schweinegrippe Erreger) abzutöten. Als Ausgangsstoff für die Gel-Matrix kann dabei Wasser mit Guarkernmehl, Karrageen oder Johannisbrotkernmehl dienen. Als oberflächenaktiver Stoff zur Verbesserung der Löslichkeit des Reinigungsgels kann Natriumlaurylsulfat verwendet werden. Als kationischer oberflächenaktiver Stoff zur Desinfektion kann Alkohol oder Didecyldimethylammoniumchlorid verwendet werden. Glycerin und Propylenglycol können als Feuchthaltemittel bzw. zur Bewahrung der Feuchtigkeit eingesetzt werden. Diese Angaben zeigen lediglich einige der Realisierungsmöglichkeiten für die Zusammensetzung des Reinigungsgels. Sonderentwicklungen für einen konkreten Bedarf, also z.B. mit spezifisch wirkenden zusätzlichen Inhaltsstoffen, sind natürlich ebenfalls möglich. Beispielsweise ist es ohne weiteres auch möglich, das von der Anmelderin entwickelte "Cyber Clean" Produkt einzusetzen, das auch vom Robert-Koch Institut als Reinigungsmasse für medizinische Anwendungen getestet und zertifiziert worden ist.

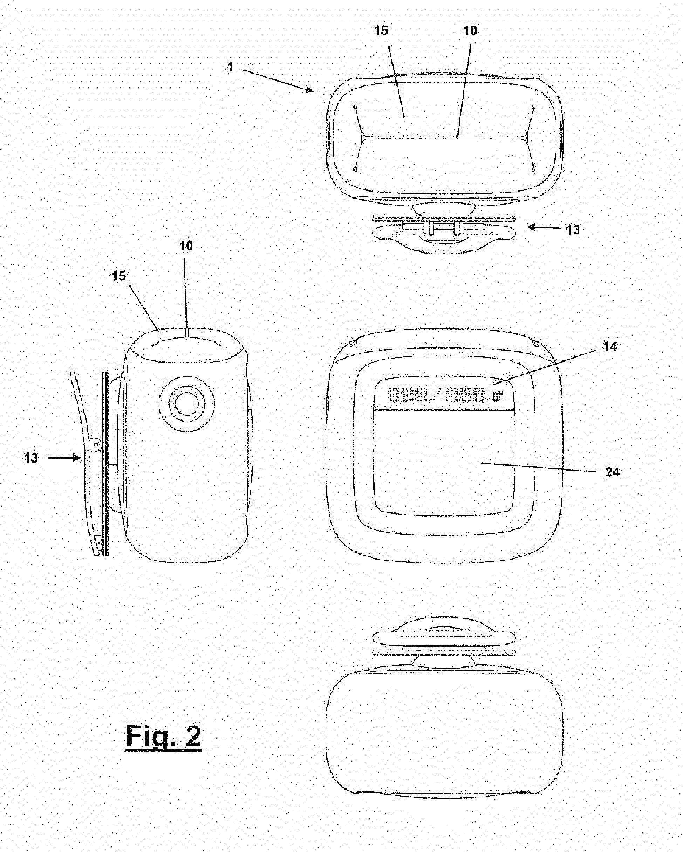

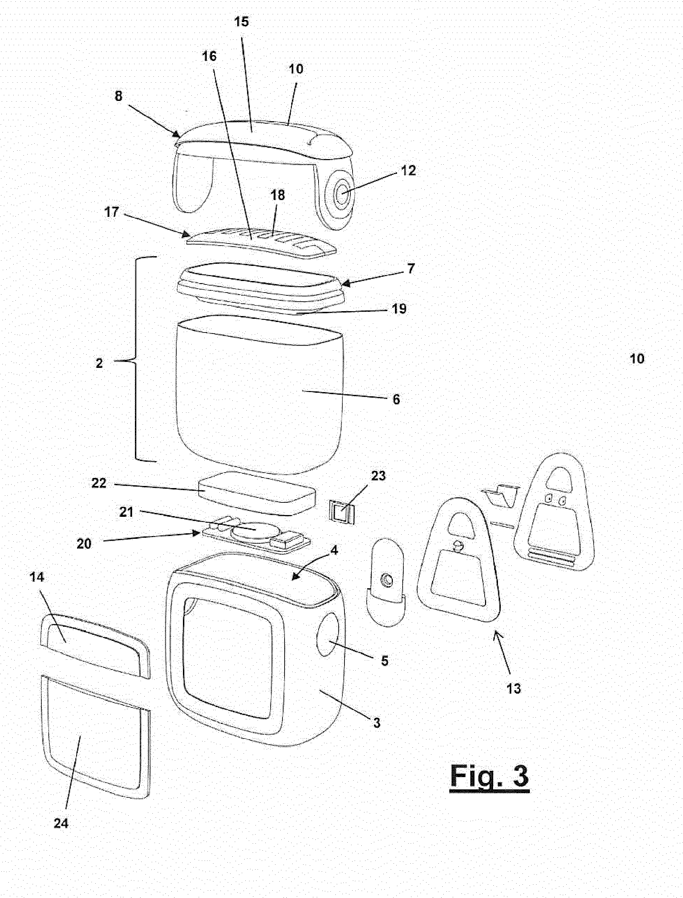

Die Fig. 2 zeigt vier Ansichten der Stethoskopreinigungsvorrichtung 1, nämlich in einer Ansicht von oben, zwei Seitenansichten und einer Ansicht von unten. Deutlich erkennbar ist hier, dass die Stethoskopreinigungsvorrichtung 1 an einer ersten Längsseite ein Befestigungsmittel in der Form einer Befestigungsklemme 13 hat, mit der sie beispielsweise an der Brusttasche eines Arztkittels befestigbar ist. Weitere Einzelheiten zur Befestigungsklemme 13 sind auch der Fig. 3 entnehmbar. An einer zweiten, der ersten Längsseite gegenüberliegenden Längsseite hat die Stethoskopreinigungsvorrichtung 1 ein Anzeigeelement 14. Lediglich angedeutet ist hier, dass das Anzeigeelement 14 die Form eines alphanumerischen Displays hat und in der Lage ist, Zahlen und Zeichen darzustellen, beispielsweise die Information, dass die Stethoskopreinigungsvorrichtung 1 bereits n-mal gebraucht worden ist und dass eine maximale Gebrauchsanzahl m zur Verfügung steht, bevor die Reinigungspatrone 1 wegen Erschöpfung der Reinigungskraft ersetzt werden muss.

Die Fig. 3 zeigt eine Explosionszeichnung der Stethoskopreinigungsvorrichtung 1. Dargestellt sind die wesentlichen zum Verständnis notwendigen Teilkomponenten des vorliegenden Ausführungsbeispiels. Nachfolgend wird auf bisher noch nicht beschriebene Teilkomponenten eingegangen:

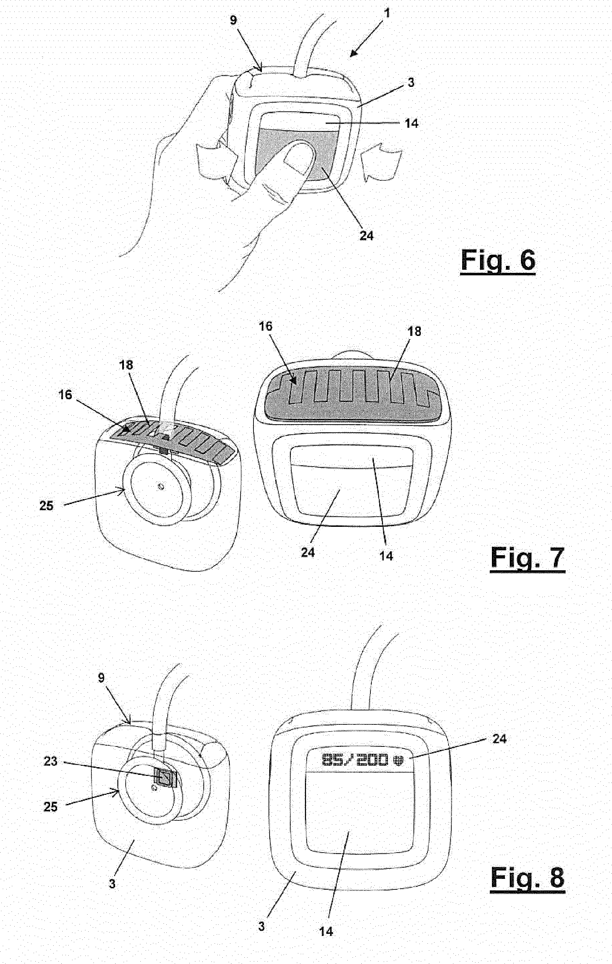

Die Lippendichtung 9 ist vorzugsweise eine zweilagige Silikonlippendichtung und besteht vorzugsweise aus einer einstückig mit dem zweiten Adapterteil 8 verbundenen äusseren Lippendichtung 15 und einer inneren Lippendichtung 16, die in einem im zweiten Adapterteil 8 einsetzbaren Einsatzteil 17 angeordnet ist. Die innere Lippendichtung hat einen mäandernden Schlitz 18 um den spezifischen Formen der zu reinigenden Stethoskope besser Rechnung zu tragen und die Gelmasse besser zurückzuhalten (siehe dazu auch Fig. 7).

Das erste Adapterteil 7 hat zudem eine umlaufende, schräg in das Innere der Reinigungspatrone ragende weitere Lippe 19 aus einem elastischen Material. Auch diese weitere Lippe 19 dient ebenfalls dazu, die Gelmasse besser im Aufnahmebehälter 3 zurückzuhalten.

Am Grund des Aufnahmebehälters ist eine elektronische Schaltung 20 mit einer Batterie 21 angeordnet und mit einer Abdeckung 22 gegen Beschädigung durch Druck vom Gel-Behälter 6 geschützt. Ein Sensorelement 23 ist an einer Seitenwand des Aufnahmebehälters 3 angeordnet (siehe dazu auch Fig. 8). Das Sensorelement kann beispielsweise ein Mikroschalter, ein lichtempfindliches Element oder ein druckempfindliches Element sein, und es dient dazu, zu detektieren, ob ein Stethoskop in die Stethoskopreinigungsvorrichtung eingeführt ist. Nicht dargestellt sind hingegen die elektrischen Verbindungen, die zwischen der elektronischen Schaltung 20, dem Sensorelement 23 und dem Anzeigeelement 14 vorhanden sein müssen um die im Zusammenhang mit der Fig. 2 bereits erwähnten Anzeigefunktionen zu realisieren. Unterhalb des Anzeigeelements 14 befindet sich ein nachgiebiges eindrückbares Plattenteil 24

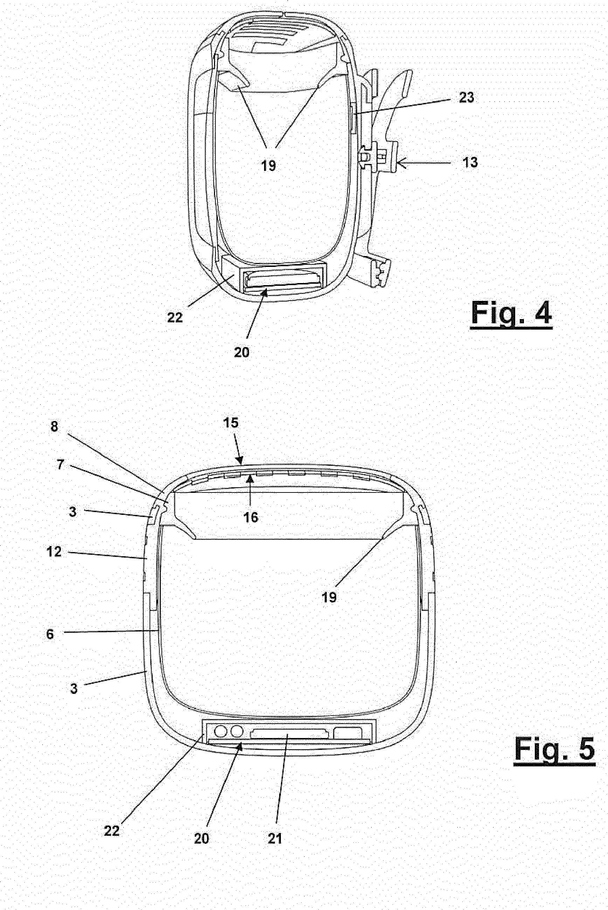

Die Figuren 4 und 5 zeigen einen Querschnitt und einen Längsschnitt durch die Stethoskopreinigungsvorrichtung 1. Besonders deutlich ersichtlich ist hier die Anordnung der inneren Lippendichtung 16, der äusseren Lippendichtung 15 und der elektronischen Schaltung 20.

Die Fig. 6 zeigt den Gebrauch der Stethoskopreinigungsvorrichtung 1 bei eingeführtem Stethoskop durch Zusammendrücken. Zu diesem Zweck wird die Stethoskopreinigungsvorrichtung 1 an einem elastischen Teilbereich des Aufnahmebehälters 3, nämlich an dem nachgiebigen Plattenteil 24, zusammengedrückt. Da der Gel-Behälter 6 der Reinigungspatrone 2 ebenfalls nachgiebig zusammendrückbar ist, gelingt es so, die Reinigungs- und Desinfektionswirkung durch intensiveren Kontakt des Stethoskops 25 mit der Reinigungsmasse noch zu verbessern.

Die Fig. 7 zeigt die Funktionsweise der inneren Lippendichtung 16 bei eingesetztem und bei entnommenem Stethoskop 25. Zur Vereinfachung der Darstellung wurde hier die äussere Lippendichtung weggelassen. Deutlich erkennbar ist der Verlauf des mäandernd Schlitzes 18, er bildet nämlich an der inneren Lippendichtung 16 beidseitig fingerartig übergreifende und wegen der Verwendung elastischen Materials (z.B. Silikon) auch lokal nachgiebige Teilstreifen die geeignet sind, den spezifischen Formen und Grössen der zu reinigenden Stethoskope besonders gut Rechnung zu tragen und die Gelmasse besser zurückzuhalten. Im linken Teil der Fig. 7 ist angedeutet, wie man sich die Verformung der nachgiebigen Teilstreifen der inneren Lippendichtung 16 vorstellen kann. Insgesamt erreicht man aber mit der vorgeschlagenen zweilagigen bzw. zweiteiligen Lippendichtung, wie in diesem Ausführungsbeispiel gezeigt, nämlich bestehend aus einer inneren und einer äusseren Lippendichtung 16, 15, besonders gute Resultate sowohl mit Hinblick auf die Rückhaltefähigkeit für die Reinigungsmasse im Gel-Behälter 6 als auch auf die Sauberkeit am Schlitz 10. Schlussendlich ist die Sauberkeit am Einführungsschlitz nicht nur von funktionaler sondern auch von ästethischer Bedeutung.

Die Fig. 8 zeigt schliesslich noch die prinzipielle Anordnung des Sensorelements 23 im Aufnahmebehälter 3 und die Positionierung des Anzeigeelements 14 am Aufnahmebehälter. Anordnung und Positionierung können in Abhängigkeit der verwendeten Komponenten natürlich verschiedenartig gestaltet sein. Bei der Verwendung eines Mikroschalters als Sensorelement 23 dürfte eine Anordnung im mittleren oder eher oberen Teil einer Seitenwand des Aufnahmebehälters 3 wohl sinnvoll sein, damit Stethoskope verschiedener Grössen sicher erkannt werden. Druckempfindliche Sensoren, wie piezoelektrische Elemente, erlauben in der Regel etwas grössere Freiheiten bezüglich der Anordnung.

Insgesamt sind aber, wie schon früher erwähnt, die elektronische Schaltung 20 und das Anzeigeelement 14 dazu ausgebildet, eine summierte Gebrauchsanzahl, also eine Zählung wie oft ein Stethoskop in die Stethoskopreinigungsvorrichtung eingesetzt worden ist, zu registrieren und anzuzeigen. Die elektronische Schaltung 20 und das Anzeigeelement 14 können auch dazu ausgebildet sein, eine vorgegebene maximale Gebrauchsanzahl zu registrieren und dann mit einem speziellen Darstellungsmodus anzuzeigen.

Schliesslich können (ohne bildliche Darstellung) im Aufnahmebehälter 3 und in der Reinigungspatrone 2 transparente Teilbereiche vorhanden sein, die es erlauben, Farbveränderungen an der Reinigungsmasse in der Reinigungspatrone von aussen zu beobachten. Zu diesem Zweck können dem Adsorptionsmittel Zusätze beigegeben werden, die mit Farbveränderung auf eine Veränderung des PH-Wertes reagieren. So ist es beispielsweise möglich, den Zusatz so zu wählen, dass das Adsorptionsmittel gelblich oder gelb erscheint, wenn der PH-Wert desselben in einem Bereich von 6.5 bis 7.5 liegt, und gräulich oder schwarz, wenn der PH-Wert ausserhalb dieses Bereiches liegt. Auf diese Weise kann die Lebensdauer bzw. die verbleibende Wirksamkeit des Adsorpionsmittels visuell und ohne Zuhilfenahme von elektronischen Mitteln angezeigt werden. Voraussetzung ist dann lediglich, dass die erwähnten transparenten Teilbereiche in geeigneter Form vorhanden und auch gut einsehbar sind.

Zurückkehrend zur Fig. 1 erfolgt das Auswechseln einer Reinigungspatrone auf die nachstehende Weise:
- die Darstellung A zeigt die Stethoskopreinigungsvorrichtung 1 zur Reinigung und Desinfektion von Stethoskopen in gebrauchsfertigem Zustand. Zum Auswechseln der Reinigungspatrone 2 werden die in den Ausnehmungen 5 befindlichen Nocken 12 zusammengedrückt
- die Darstellung B zeigt, dass die Reinigungspatrone 2 nunmehr mitsamt dem aufgesetzten zweiten Adapterteil 8 aus dem Aufnahmebehälter 3 herausgehoben werden kann.
- Die Darstellung C zeigt, dass das zweite Adapterteil 8 anschliessend vom ersten Adapterteil 7, das seinerseits fest mit dem Gel-Behälter 6 der Reinigungspatrone 2 verbunden ist, gelöst werden kann. Die verbrauchte Reinigungspatrone 2 (grau schattiert), kann einem Recyclingprozess oder der Entsorgung zugeführt werden. Anstelle der verbrauchten Reinigungspatrone kann (nach dem Abziehen der früher erwähnten Schutzfolie) eine neue unverbrauchte Reinigungspatrone eingesetzt werden und die Stethoskopreinigungsvorrichtung 1 kann in umgekehrter Reihenfolge wieder zusammengesetzt werden.

### Bezugszififernliste:

- 1: Stethoskopreinigungsvorrichtung
- 2: Reinigungspatrone
- 3: Aufnahmebehälter
- 4: Aufnahmeöffnung
- 5: Ausnehmung
- 6: Gel-Behälter
- 7: erstes Adapterteil
- 8: zweites Adapterteil
- 9: Lippendichtung
- 10: Schlitz
- 11: Lasche
- 12: Nocken
- 13: Befestigungsklemme
- 14: Anzeigeelement
- 15: äussere Lippendichtung
- 16: innere Lippendichtung
- 17: Einsatzteil
- 18: mäandernder Schlitz
- 19: weitere Lippe
- 20: elektronische Schaltung
- 21: Batterie
- 22: Abdeckung
- 23: Sensorelement
- 24: nachgiebiges Plattenteil
- 25: Stethoskop

## Patentansprüche

1. Stethoskopreinigungsvorrichtung (1) zur Reinigung und Desinfektion von Stethoskopen, umfassend einen Aufnahmebehälter (3) mit einer Aufnahmeöffnung (4), in dem eine eine Reinigungsmasse enthaltende Reinigungspatrone (2) auswechselbar gehalten ist, wobei die Reinigungsmasse ein Reinigungsgel ist,
**dadurch gekennzeichnet, dass**
- ein erstes an der Reinigungspatrone (2) angebrachtes Adapterteil (7) mit einem aufsetzbaren zweiten Adapterteil (8) einrastend verbindbar ist, dass
- das zweite Adapterteil (8) mit dem Aufnahmebehälter (3) einrastend verbindbar ist, und dass
- Das zweite Adapterteil (8) eine Lippendichtung (9) aus einem elastischen Material aufweist.

2. Stethoskopreinigungsvorrichtung nach Patentanspruch 1, **dadurch gekennzeichnet, dass** die Lippendichtung (9) eine zweilagige Silikonlippendichtung ist, mit einer äusseren, einstückig mit dem zweiten Adapterteil (8) verbundenen äusseren Lippendichtung (15) und einer inneren Lippendichtung (16) die in einem im zweiten Adapterteil (8) einsetzbaren Einsatzteil (17) angeordnet ist.

3. Stethoskopreinigungsvorrichtung nach Patentanspruch 1 oder 2, **dadurch gekennzeichnet, dass** das erste Adapterteil (7) eine umlaufende, schräg in das Innere der Reinigungspatrone (2) ragende weitere Lippe (19) aus einem elastischen Material aufweist.

4. Stethoskopreinigungsvorrichtung nach einem der Patentansprüche 1 bis 3, **dadurch gekennzeichnet, dass** die Reinigungspatrone (1) im Wesentlichen elastisch zusammendrückbar ist und dass der Aufnahmebehälter (3) mindestens in einem Teilbereich ebenfalls elastisch zusammendrückbar ist, wobei die Reinigungspatrone (2) im eingesetzten Zustand über den Teilbereich des Aufnahmebehälters elastisch zusammendrückbar ist um die Reinigungs- und Desinfektionswirkung zu verbessern.

5. Stethoskopreinigungsvorrichtung nach einem der Patentansprüche 1 bis 4, **dadurch gekennzeichnet, dass** im Aufnahmebehälter (3) eine elektronische Schaltung (20) und ein Sensorelement (23) angeordnet sind und dass am Aufnahmebehälter (3) ein Anzeigeelement (14) vorhanden ist.

6. Stethoskopreinigungsvorrichtung nach Patentanspruch 5, **dadurch gekennzeichnet, dass** das Sensorelement (23) dazu ausgebildet ist, zu detektieren, ob ein Stethoskop in die Stethoskopreinigungsvorrichtung (1) eingesetzt ist, und wobei das Sensorelement ein Mikroschalter, ein lichtempfindliches Element oder ein druckempfindliches Element ist.

7. Stethoskopreinigungsvorrichtung nach Patentanspruch 5, **dadurch gekennzeichnet, dass** die elektronische Schaltung (20) und das Anzeigeelement (14) dazu ausgebildet sind, eine summierte Gebrauchsanzahl, also eine Zählung wie oft ein Stethoskop in die Stethoskopreinigungsvorrichtung eingesetzt worden ist, zu registrieren und anzuzeigen.

8. Stethoskopreinigungsvorrichtung nach Patentanspruch 7, **dadurch gekennzeichnet, dass** die elektronische Schaltung (20) und das Anzeigeelement (14) dazu ausgebildet sind, eine vorgegebene maximale Gebrauchsanzahl zu registrieren und mit einem speziellen Darstellungsmodus einen Ersatzbedarf für die Reinigungspatrone (2) anzuzeigen.

9. Stethoskopreinigungsvorrichtung nach einem der Patentansprüche 1 bis 4, **dadurch gekennzeichnet, dass** im Aufnahmebehälter (3) und in der Reinigungspatrone (2) transparente Teilbereiche vorhanden sind, die es erlauben Farbveränderungen an der Reinigungsmasse in der Reinigungspatrone von aussen zu beobachten.

10. Stethoskopreinigungsvorrichtung nach einem der Patentansprüche 1 bis 9, **dadurch gekennzeichnet, dass** die Stethoskopreinigungsvorrichtung mit Befestigungsmitteln versehen ist, um leicht zugänglich an einem Bekleidungsstück angebracht zu werden.
